# EUROPEAN PATENT APPLICATION

(11) **EP 2 574 927 A1**
(43) Date of publication of application: **03.04.2013**
(21) Application number: 12184647.1
(22) Date of filing: 17.09.2012
(51) Int. Cl.: G01N 33/543, G01N 33/58

(54) **Method for measuring substance to be measured using fluorescent particles, test subsance measurement chip, and test subsance measurement kit**

(30) Priority: 28.09.2011 JP 2011211923; 30.08.2012 JP 2012189846
(71) Applicant: Fujifilm Corporation, Minato-ku Tokyo 106-8620 (JP)
(72) Inventor: Watanabe, Yuya, Ashigarakami-gun Kanagawa, 258-8577 (JP); Kasagi, Noriyuki, Ashigarakami-gun Kanagawa, 258-8577 (JP)
(74) Representative: HOFFMANN EITLE

(57) **Abstract**

A method for detecting a test substance including (1) reacting fluorescent particles and a test substance by bringing the test substance and the fluorescent particles into contact with a test area and a control area present on a substrate, (2) removing the unreacted fluorescent particles, (3) measuring the fluorescence of the fluorescent particles of the test area and the control area, and (4) correcting a fluorescence signal value in the test area using a fluorescence signal value in the control area, wherein the fluorescent particles are binding substance-labeling fluorescent particles in which one or more of a first binding substance which can bind to the test substance, and a third binding substance which can bind to a second binding substance and which does not bind to the test substance are bound, and the second binding substance which can bind to the first binding substance is immobilized on the control area.

## Description

### BACKGROUND OF THE INVENTION

1. Field of the Invention

The present invention relates to a method for detecting a substance to be detected using fluorescent particles.

2. Description of the Related Art

Generally, a fluorescence detection method has been widely used as a highly sensitive and simple measuring method to quantify proteins, enzymes, inorganic compounds, and the like. This fluorescence detection method is a method in which a sample presumed to include a substance to be detected (a test substance) which emits fluorescence by being excited by light of a specific wavelength is irradiated with excitation light of the specific wavelength, and the presence of a test substance is confirmed by detecting the fluorescence at that time. In addition, if the test substance is not a phosphor, a method, in which a substance specifically binding to a test substance by being labeled with a fluorescent dye is brought into contact with a sample, and then, fluorescence is detected in the same manner described above, therefore, this binding, that is, the presence of the test substance is confirmed, has also been widely used.

In the fluorescence detection method such as this, a method which uses the effect of an electric field enhancement due to plasmon resonance to improve the sensitivity of detection is known. In such a method, a sensor chip is prepared in which a metal layer is provided in a predetermined region on a transparent support in order to generate a plasmon resonance, and excitation light is incident at a predetermined angle of total reflection or larger from the opposite side of the metal layer forming surface of the support with respect to the interface between a support and a metal film. Surface plasmon is generated on the metal layer by the irradiation of excitation light such as this. By the action of an electric field enhancement due to the occurrence of surface plasmon, the signal/noise ratio (S/N ratio) is improved by enhancing the fluorescence. In a fluorescence detection method by surface plasmon excitation (hereinafter, referred to as an "SPF method"), when compared with a fluorescence detection method by epi excitation (hereinafter, referred to as an "epi-fluorescence method"), signal enhancement of approximately 10 times is obtained, therefore, a measurement with high sensitivity is possible.

For example, in a method for detecting an optical signal to determine the amount of a test substance disclosed in JP2010-190880A, a sensor chip having a sensor unit with a metal layer provided in a predetermined region of the surface of a dielectric plate is prepared, and a sample is brought into contact with the sensor unit of the sensor chip. A binding substance, in which an amount of photoresponsive labeling substance depending on the amount of a test substance included in the sample is applied, binds to the sensor unit by such a contact. Subsequently, the amount of the test substance is determined by irradiating excitation light on a predetermined area, and by detecting the light from the photoresponsive labeling substance generating within the field of the electric field enhancement formed on the metal layer. In addition, in this method, a plurality of photoresponsive materials may be included and used as photoresponsive labeling substances in order to prevent metal quenching which occurs when the photoresponsive material is closer to the metal layer due to light transparent materials which allow the light generated from the photoresponsive material to pass through.

In addition, in an assay method disclosed in JP1998-90268A (JP-H10-90268A), extending the concentration range of a test substance which can be quantified has become possible by an immunoassay using a plurality types of antibodies against a test substance.

### SUMMARY OF THE INVENTION

In a related immunodiagnostic system using an SPF method, a method has been adopted in which variations in measurement results are corrected based on the correction factor obtained by dividing the signal value of a test area reacting to the object to be measured by the signal value of a control area proportional to the amount of reagent in order to reduce variability of the amount of reagent included in the product or the like. For this reason, there has been a problem in that variations are not properly corrected even when such a correction coefficient is used if the signal change due to temperature changes and the like changes independently to a signal value of a test area and the signal value of a control area. In general, adjusting the rate of signal change due to temperature changes and the like has been carried out by changing the amount of an antibody fixed on the substrate or by varying the amount of an antibody fixed on the label. Specifically, temperature dependence can be increased from an adjustment by reducing the amount of an antibody fixed on the substrate. However, the rate of signal change due to temperature changes sometimes may not be adjusted even when the amount of an antibody fixed on the substrate or the amount of an antibody fixed on the label is changed. The challenge of the present invention is to solve the problems of the related art described above. In other words, the challenge of the present invention is to provide a detection method for a test substance with small differences of temperature dependence between the signal values of a test area and the signal value of a control area in a fluorescence assay using a correction system adjusting the rate of signal change due to temperature changes and the like.

As a result of extensive studies to solve the above problems, the inventors have found that reducing the differences of temperature dependence between the signal values of a test area and the signal value of a control area is possible by carrying out a fluorescence assay using fluorescent particles in which a first binding substance which can bind to a test substance, and a third binding substance which can bind to a second binding substance capable of binding to the first binding substance and does not bind to a test substance are bound. The present invention has been completed based on these findings.

In other words, according to the present invention, provided is a method for measuring a test substance including (1) reacting fluorescent particles and the test substance by bringing the test substance and the fluorescent particles into contact with a test area and a control area present on a substrate, (2) measuring the fluorescence of the fluorescent particles of the test area and the control area, and (3) correcting a fluorescence signal value in the test area using a fluorescence signal value in the control area, wherein the fluorescent particles are binding substance-labeling fluorescent particles in which (i) one or more of a first binding substance which can bind to the test substance, and (ii) a third binding substance which can bind to a second binding substance and does not bind to the test substance are bound, and wherein the second binding substance which can bind to the first binding substance is immobilized on the control area.

It is preferable that, in step (2), the fluorescence signal value in each area be determined by measuring fluorescence of the binding substance-labeling fluorescent particles in each of the test area and the control area, and the fluorescence signal value in the test area be corrected using the fluorescence signal value in the control area.

It is preferable that the test substance or an analogous compound of the test substance having an epitope for the first binding substance which binds to the test substance or the fluorescent particles be immobilized on the test area.

It is preferable that, in step (1), the test substance brought into contact with the substrate and the test substance or the analogous compound of the test substance having an epitope binding to the test area of the substrate be made to compete for the binding substance-labeling fluorescent particles.

It is preferable that the first binding substance be an antibody which can bind to the test substance, and the second binding substance immobilized on the control area be an antibody which can bind to the antibody which is the first binding substance and to the third binding substance which does not bind to the test substance, respectively.

It is preferable that the third binding substance be an antibody which can bind to the second binding substance and does not bind to the test substance.

It is preferable that the fluorescent particles be fluorescent latex particles.

It is preferable that, in step (2), the fluorescence be measured using surface plasmon fluorescence measurement or epi-fluorescence measurement.

In addition, according to the present invention, provided is a test substance measurement chip used in measuring a test substance including, a test area and a control area on a substrate, wherein fluorescent particles and a test substance are reacted by bringing each area into contact with the test substance and a test sample containing the fluorescent particles, the fluorescence of the fluorescent particles of each area is measured, and a fluorescence signal value in the test area is corrected using a fluorescence signal value in the control area, wherein the fluorescent particles are binding substance-labeling fluorescent particles in which (i) one or more of a first binding substance which can bind to the test substance, and (ii) a third binding substance which can bind to a second binding substance and which does not bind to the test substance are bound, and wherein the second binding substance which can bind to the first binding substance is immobilized on the control area.

In addition, according to the present invention, provided is a test substance measurement kit constituted of a test substance measurement chip including, a test area and a control area on a substrate and used in measuring a test substance, by reacting fluorescent particles and the test substance by bringing each area into contact with the test substance and a test sample containing the fluorescent particles, measuring the fluorescence of the fluorescent particles of each area, and correcting a fluorescence signal value in the test area using a fluorescence signal value in the control area, and fluorescent particles used for the test sample, wherein the fluorescent particles are binding substance-labeling fluorescent particles in which (i) one or more of a first binding substance which can bind to the test substance, and (ii) a third binding substance which can bind to a second binding substance and which does not bind to the test substance are bound, and wherein the second binding substance which can bind to the first binding substance is immobilized on the control area.

According to the present invention, a method for detecting a test substance using fluorescence which can reduce the differences of temperature dependence between signal values of a test area and a signal value of a control area in a fluorescence assay using a correction system adjusting the rate of signal change due to temperature changes, and the like is provided.

### BRIEF DESCRIPTION OF THE DRAWINGS

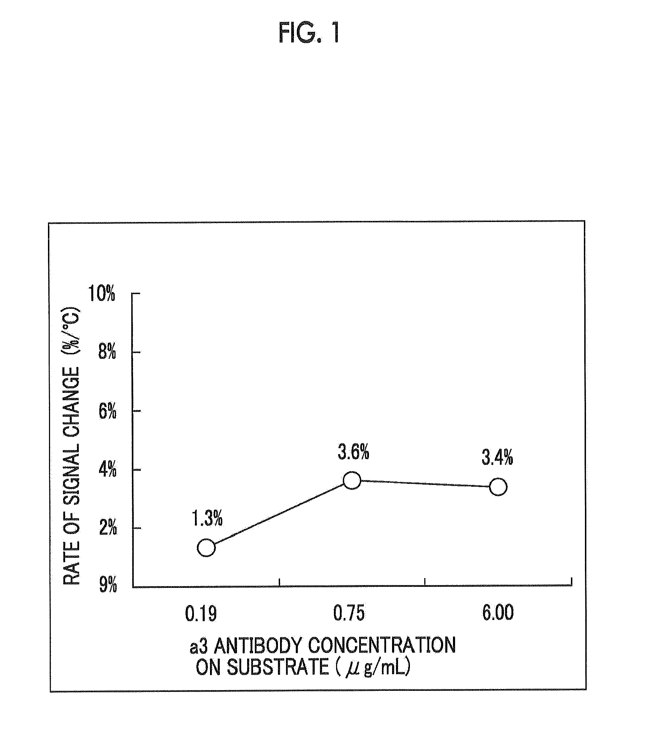

Fig. 1 shows a relationship between the concentration of an antibody immobilized on a substrate which is a second binding substance and a rate of signal change when the antibody concentration and the temperature at the time of a measurement are varied in a control area in which the second binding substance capable of binding to an antibody which is the first binding substance is immobilized in a case of fluorescent particles in which one type of antibody which is a first binding substance is immobilized is used.

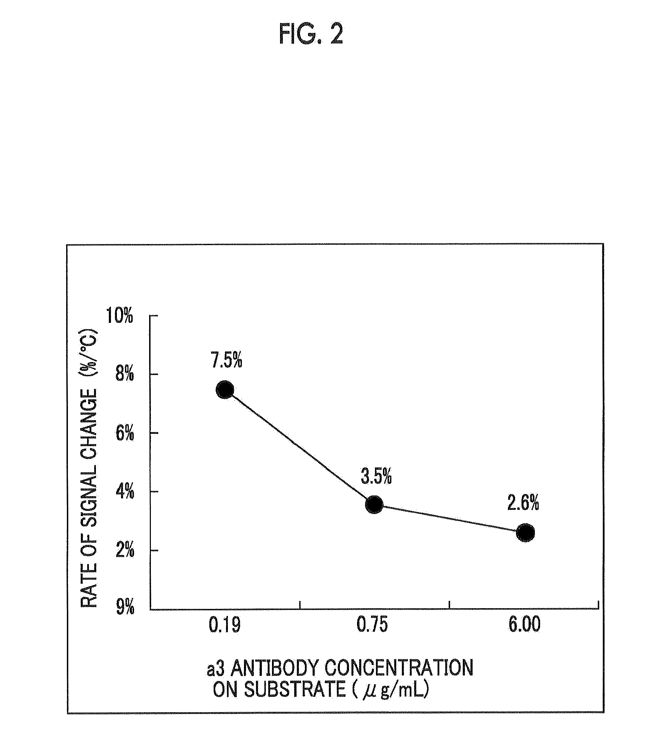

Fig. 2 shows a relationship between the concentration of an antibody immobilized on a substrate which is a second binding substance and a rate of signal change when the antibody concentration and the temperature at the time of a measurement are varied in a control area in which the second binding substance capable of binding to an antibody which is the first binding substance is immobilized in a case of fluorescent particles in which two types of antibodies (an antibody against a test substance; that is an antibody which is a first binding substance, and an antibody against substances other than the test substance; that is, an antibody which is a third binding substance) are immobilized is used.

### DESCRIPTION OF THE PREFERRED EMBODIMENTS

Hereinafter, the present invention will be described in more detail.

The method for measuring a test substance of the present invention is a method for measuring a test substance including (1) reacting fluorescent particles and a test substance by bringing the test substance and the fluorescent particles into contact with a test area and a control area present on a substrate, (2) measuring the fluorescence of the fluorescent particles of the test area and the control area, and (3) correcting a fluorescence signal value in the test area using a fluorescence signal value in the control area, wherein the fluorescent particles are binding substance-labeling fluorescent particles in which (i) one or more of a first binding substance which can bind to the test substance, and (ii) a third binding substance which can bind to a second binding substance and which does not bind to the test substance are bound, and the second binding substance which can bind to the first binding substance is immobilized on the control area.

In step (2), the fluorescence signal value in each area is determined by measuring fluorescence of the binding substance-labeling fluorescent particles in each of the test area and the control area, and the fluorescence signal value in the test area is corrected using the fluorescence signal value in the control area. One example of the methods which corrects the fluorescence signal value may include a method in which the fluorescence signal value in the test area is divided by the fluorescence signal value in the control area, however, the method is not limited to this, and for example, the fluorescence signal value in the test area may be corrected by a conversion factor corresponding to the fluorescence signal value in the control area calculated from a related equation, and the correction may be carried out by subtracting the fluorescence signal value in the control area from the fluorescence signal value in the test area.

(Fluorescent Particles)

As the fluorescent particles used in the present invention, particles colored with fluorescence which are commonly used in immune reactions may be used, and for example, fluorescent polymer particles such as fluorescent polystyrene beads or fluorescent glass particles such as fluorescent glass beads may be used. Specific examples of the material of the fluorescent polymer particles are synthetic polymers such as polymers using monomers such as styrene, methacrylic acid, glycidyl (meth)acrylate, butadiene, vinyl chloride, vinyl acetate acrylate, methyl methacrylate, ethyl methacrylate, phenyl methacrylate or butyl methacrylate, or copolymers using two or more monomers, and a latex in which these are uniformly suspended are preferable. In addition, organic polymer powders or inorganic material powders, microorganisms, blood cells or cell membrane fragments, liposomes, or the like, in addition to these may be included.

If the latex particles are used, specific examples of the materials of the latex may include polystyrene, a styrene-acrylic acid copolymer, a styrene-(meth)acrylic acid copolymer, a styrene-glycidyl (meth)acrylate copolymer, a styrene-styrene sulfonic acid salt copolymer, a methacrylic acid copolymer, an acrylic acid copolymer, an acrylonitrile-butadiene-styrene copolymer, a vinyl chloride-acrylate copolymer, a polyvinyl acetate acrylate, or the like. As the latex, a copolymer containing at least a styrene as a monomer is preferable, and a copolymer of styrene, and acrylic acid or methacrylic acid is particularly preferable. Preparation methods of the latex are not particularly limited, and any polymerization method may be used. However, immobilization of an antibody becomes difficult in the presence of a surfactant at the time of an antibody labeling, therefore, in the preparation of the latex, an emulsion polymerization is preferable, which is an emulsifier-free emulsion polymerization, that is, without using an emulsifier such as a surfactant.

If the latex itself obtained by polymerization is fluorescent, it can be used as the fluorescent latex particles as it is. If the latex obtained by polymerization is non-fluorescent, the fluorescent latex particles can be prepared by adding a fluorescent material (such as a fluorescent dye), to the latex. In other words, the fluorescent latex particles can be prepared by, for example, adding a fluorescent dye to the solution of latex particles containing water and a water-soluble organic solvent followed by stirring.

An average particle diameter of the fluorescent particle is different depending on the materials of the particle, the concentration range of test substance quantifiable, and measuring equipment, however, is preferably in a range of 0.001 to 10 µm (more preferably 0.001 to 1 µm). Liposomes or microcapsules containing a fluorescent dye may also be used as the fluorescent particles. A fluorescent chromophore is not particularly limited as long as it is excited by an absorption of ultraviolet light and the like, and is released when returning to the ground state, however, for example, a fluorescent chromophore such as yellow-green (excitation wavelength of 505 nm/emission wavelength of 515 nm, hereinafter the same), blue (350 to 356 nm/415 to 440 nm), red (535 to 580 nm/575 to 605 nm), orange (540 nm/560 nm), red-orange (565 nm/580 nm), crimson (625 nm/645 nm), and dark red (660 nm/680 nm) may be used. The fluorescent particles which emit such fluorescence are, for example, available from Life Technologies Corporation (formerly Invitrogen Corporation), and are commercially available under the trade name of FluoSpheres (registered trademark) from the company.

(Method of detecting Average Particle Diameter)

The average particle diameter of the fluorescent particles used in the present invention may be measured using a commercially available particle size distribution analyzer, or the like. As methods of measuring particle size distribution, an optical microscopy method, a confocal laser microscopy method, an electron microscopy method, an atomic force microscopy method, a static light scattering method, a laser diffraction method, a dynamic light scattering method, a centrifugal sedimentation method, an electrical pulse measurement method, a chromatography method, an ultrasonic attenuation method, and the like, are known, and devices that support each principle are commercially available.

Dynamic light scattering method is preferably used in the present invention considering the particle range and readiness of measurement. As the commercially available measuring devices using dynamic light scattering, a nano track UPA (Nikkiso Co., Ltd.), a dynamic light scattering-type particle size distribution measuring device LB-550 (Horiba, Ltd.), a dense system particle size analyzer FPAR-1000 (Otsuka Electronics Co., Ltd.) and the like, may be included, and in the present invention, an average particle diameter is determined as the value of a median diameter (d = 50) measured at a measurement temperature of 25°C.

(Test Substance)

The type of the test substance to be detected in an assay method of the present invention is not particularly limited, however, may include, for example, cortisol, IGF-I, IGFBP-3, LH, TSH, ADH, GH, GH in urine, ACTH, prolactin, FSH, TBG, TSAb, T4, anti-TPO antibodies, microsome antibodies, anti-thyroglobulin antibodies, TSH, thyroglobulin, T3, fT4, fT3, 1,25-(OH)2 vitamin D, NTx, Intact PINP, osteocalcin, calcitonin, BAP, deoxypyridinoline, PTH, PTHrP, 5-HIAA, HVA, L-DOPA, MHPG, VMA, catecholamine, serotonin, metanephrines, 11-deoxycortisol, 17-KGS, 17-OH pregnenolone, aldosterone, androsterone, androstenedione, 11-OHCS, corticosterone, cortisone, DOC, DHEA-S, pregnenolone, 5α-dihydrotestosterone, HCG-β subunit, E2, E3, estrogen, E1, HCG, testosterone, pregnanediol, pregnanetriol, progesterone, CPR, VIP, insulin, gastrin, glucagon, an anti-GAD antibody, an anti-IA-2 antibody, an anti-insulin antibody, cardiac troponin T, ventricular muscle myosin light chain I, H-FABP, HANP, BNP, NT-proBNP, myoglobin, or the like. One example of the test substance particularly preferable is cortisol.

(Binding Substance)

In the present invention, the binding substance-labeling fluorescent particles obtained by binding one or more of the first binding substance which can bind to the fluorescent particles is used. The first binding substance which can bind to the test substance may be, for example, a compound having an affinity for the test substance such as an antibody against the test substance (an antigen), an antigen against the test substance (an antibody), an aptamer against the test substance (protein, low-molecular-weight compounds, avidin, biotin, or the like), and is not particularly limited.

In addition, the third binding substance which can bind to the second substance (that is, "the second binding substance which can bind to the first binding substance capable of binding to the test substance" immobilized on the control area) described later in the present specification and does not bind to the test substance further binds to the binding substance-labeling fluorescent particles used in the present invention.

(First Binding Substance)

Preferable examples of the first binding substance used in the method of the present invention are antigens, antibodies, or a complex of these, however, are not limited to these. For example, if the first binding substance is an antibody, for example, antiserum prepared from the serum of immunized animals by the test substance, immunoglobulin fractions purified from the antiserum, monoclonal antibodies obtained by cell fusion using spleen cells of immunized animals by the test substance, and fragments thereof [for example, F(ab')2, Fab, Fab', or Fv], or the like may be used as the antibody having a specificity for the test substance. Preparation of these antibodies may be carried out using conventional methods. In addition, the antibody may be a modified antibody such as a chimeric antibody, a commercially available antibody, or an antibody prepared from an animal serum or a culture supernatant using well-known methods.

Antibodies may be used regardless of animal species thereof and subclasses and the like. For example, antibodies which can be used in the present invention are antibodies derived from living organisms in which an immune reaction may occur such as mice, rats, hamsters, goats, rabbits, sheep, cows or chickens, specifically, mouse IgG, mouse IgM, rat IgG, rat IgM, hamster IgG, rabbit IgG, rabbit IgM, goat IgG, goat IgM, sheep IgG, sheep IgM, bovine IgG, bovine IgM, bird IgY, or the like, and both polyclonal or monoclonal antibodies may be applied. Fragmented antibodies are molecules derived from complete type antibodies having at least one antigen-binding site, and include specifically, Fab, F(ab')2 or the like. These fragmented antibodies are molecules obtained using an enzyme or chemical treatment, or using genetic engineering techniques.

Methods for immobilizing the first binding substance such as an antibody or an antigen to the particles, for example, are disclosed in JP2000-206115A or described in the protocols attached to Molecular Probes, Inc. FluoSpheres a polystyrene microsphere F8813 (registered trademark), and all well-known methods for preparing reagents for immune agglutination reaction may be used. In addition, as the principles of immobilizing the antibody on the particles as the first binding substance, any principle of physical adsorption and a chemical bonding by covalent bonding may be employed. As a blocking agent covering the particle surface in which an antibody is coated after the antibody is immobilized to the particles, well-known substances, for example, BSA (bovine serum albumin) or skim milk, casein, ingredients derived from soybeans, ingredients derived from fish, polyethylene glycol or the like, or commercially available blocking agents for the immune reaction including these substances or substances of the same nature as these, or the like, may be used. These blocking agents may also be pretreated such as by partial denaturation by heat or acid and alkali as necessary.

(Second Binding Substance)

In the present invention, the second binding substance which can bind to the first binding substance capable of binding to the test substance is immobilized on the control area. In other words, as the second binding substance immobilized on the control area, a binding substance capable of binding to one or more the first binding substance which binds to the fluorescent particles may be used. As the second binding substance capable of binding to the first binding substance, for example, antibodies against the binding substance (an antibody), proteins (Protein A or Protein G) binding to the binding substance (an antibody) or the like, and compounds having an affinity for the first binding substance may be preferably used. Furthermore, compounds in which part of one or more of the first binding substance which binds to the fluorescent particles has a ligand-nonligand relationship with the second binding substance may be preferably used. Methods for immobilizing the second binding substance such as an antibody on a substrate are described in, for example, Tech Notes Vol. 2 to 12 provided by Nunc, and the like, and all well-known methods preparing general ELISA reagents may be used. In addition, surface modification by disposing a self-assembled monolayer (SAM) on the substrate may also be performed, and as the principles of immobilizing the antibody on the substrate as the second binding substance, any principle of physical adsorption and chemical bonding by covalent bonding may be employed. As a blocking agent covering the substrate surface in which the antibody is coated after the antibody is immobilized on the substrate, well-known substances, for example, BSA (bovine serum albumin) or skim milk, casein, ingredients derived from soybeans, ingredients derived from fish, polyethylene glycol or the like, or commercially available blocking agents for the immune reaction including these substances or substances of the same nature as these, or the like, may be used. These blocking agents may also be pretreated such as by partial denaturation by heat or acid and alkali as necessary.

(Third Binding Substance)

As the third binding substance which can bind to the second binding substance and does not bind to the test substance, antibodies that bind to substances other than the test substance may be used, and compounds which can capture the second binding substance such as fragments thereof [for example, F(ab')2, Fab, Fab', Fv or Fc] may be used as well.

(Immobilization of Antibody to Fluorescent Particles)

Specific methods to immobilize the antibody on the particles will be exemplified below. An antibody solution adjusted to a concentration of 0.01 to 20 mg/mL is added to a solution in which particles are dispersed with the solids concentration to be 0.1 to 10% by mass, and is mixed. Stirring is continued for 5 minutes to 48 hours under the conditions of a temperature of 4 to 50°C. Subsequently, antibodies which do not bind to the particles included in the solution are removed sufficiently by separating the particles from the solution using methods other than centrifugation. After that, an operation in which the particles are washed with a buffer solution is repeated 0 to 10 times. After performing the operation which mixes the particles and the antibodies and binds the antibodies to the particles, it is preferable that the portion in which the antibody of the particle surface does not bind be protected by a blocking agent such as the components which do not participate in the antigen-antibody reaction, preferably a protein, and more preferably BSA (bovine serum albumin), Block Ace, skim milk casein, and the like.

In the present invention, one or more of the first binding substance which can bind to the test substance and the third binding substance which can bind to the second substance and does not bind to the test substance are mixed in advance, and then bound to the particle using the method described above, or one or more of the first binding substance which can bind to the test substance and the third binding substance which can bind to the second substance and does not bind to the test substance are sequentially bound to the particle, however, being mixed in advance and then bound to the particle is preferable.

A stabilizing agent may be added if necessary when antigens, antibodies or the like are immobilized on the particles. The stabilizing agent is not particularly limited and synthetic or natural polymers such as sucrose or polysaccharides may be used as long as they stabilize antigens or antibodies, and commercially available products such as Immunoassay Stabilizer (Life Technologies Corporation (formerly ABI)) may also be used.

(Assay Form)

An assay method of the present invention includes (1) reacting the fluorescent particles and the test substance by bringing the test substance and the fluorescent particles into contact with the test area and the control area present on the substrate, (2) measuring the fluorescence of the fluorescent particles of the test area and the control area, and (3) correcting the fluorescence signal value in the test area using the fluorescence signal value in the control area.

The detection method of the test substance according to the present invention is to be construed as the broadest concept including a detection of the presence or absence of the test substance, a measurement of the amount of the test substance (that is, quantitative), or the like, and extensively includes an immunological measurement commonly known as a method of measurement using a substrate.

(Contacting Step)

As the method of contacting the binding substance-labeling fluorescent particles and the test substance on the substrate in a sandwich method and a competitive method, for example, general ELISA assay methods described in p.258 of the basic experimental information provided by Sigma-Aldrich (that is, a method of contacting liquid in which fluorescent particles and a test substance are dispersed or dissolved on a substrate by adding the liquid) may be used. By providing the contact place in a microchannel, it is also possible that the liquid in which the fluorescent particles and the test substance are dispersed or dissolved be made to flow and come into contact. Hereinafter, as specific embodiments of the assay methods of the present invention, a sandwich method and a competitive method will be described, however, the present invention is not limited to these.

(Sandwich Method)

In the sandwich method, while not particularly limited, for example, the test substance may be measured by the following procedure. First, the first binding substance which has a specificity for the test substance, the second binding substance which can bind to the third binding substance, and the third binding substance which does not bind to the test substance, but which does bind to the second binding substance are prepared in advance. Subsequently, the binding substance-labeling fluorescent particles are prepared by binding the first binding substance and the third binding substance to the fluorescent particles. In addition, the second binding substance is immobilized on the substrate and this is made to be a control area. If the sandwich method is used in the present invention, a fourth binding substance which binds to the test substance, but which does not bind to the first binding substance and the third binding substance is prepared separately, is immobilized on the substrate, and this is made to be a test area. Next, a test sample (or an extract thereof) which may contain the test substance, and the binding substance-labeling fluorescent particles are brought into contact with the test area and the control area on the substrate. If the test substance is present in the test sample, reaction occurs on the test area between the test substance and the first binding substance which binds to the binding substance-labeling fluorescent particles, and the test substance and the fourth binding substance on the test area (an antigen-antibody reaction when antigens and antibodies are used), and the fluorescent particles corresponding to the amount of the test substance are immobilized on the test area. On the other hand, reaction occurs on the control area between the first binding substance which binds to the binding substance-labeling fluorescent particles or the third substance, and the second binding substance immobilized on the control area, and the fluorescent particles are immobilized on the control area. In the sandwich method, after the reaction between the fourth binding substance immobilized on the test area and the test substance, and the reaction between the test substance and the first binding substance which binds to the binding substance-labeling fluorescent particles are completed, and also the reaction between the second binding substance immobilized on the control area and the first and the third binding substances on the binding substance-labeling fluorescent particles is completed, the binding substance-labeling fluorescent particles which do not bind to the areas on the two substrates are removed and washed away. Next, the signal intensity from the binding substance-labeling fluorescent particles which bind to the test area is detected and corrected using a measurement value of the signal intensity from the binding substance-labeling fluorescent particles which bind to the control area, and as a result, the exact concentration of the test substance may be measured. In addition, the fluorescence intensity and the concentration of the test substance have a positive correlation.

The fourth binding substance is not particularly limited as long as it can bind to the test substance and does not bind to the first binding substance and the third binding substance. Antibodies which do not bind to the first binding substance and the third binding substance, however, can bind to the test substance or compounds such as fragments thereof [for example, F(ab')2, Fab, Fab', Fv or Fc] may preferably be used.

(Competitive Method)

In the competitive method, while not particularly limited, for example, the test substance may be measured by the following procedure. The competition method is well known in the art as a method to detect antigens of low molecular weight compounds which may not be assayed using the sandwich method.

First, the first binding substance which has a specificity for the test substance, the second binding substance which can bind to the third binding substance, and the third binding substance which does not bind to the test substance and binds to the second binding substance are prepared in advance. Subsequently, the binding substance-labeling fluorescent particles are prepared by binding the first binding substance and the third binding substance to the fluorescent particles. Next, the second binding substance which can bind to the third binding substance is immobilized on the solid phase, for example, a substrate and this is made to be a control area. Furthermore, the test substance itself which is capable of binding to the first binding substance, or a compound having a similar site to the test substance and having the same epitope for the first binding substance as the test substance is immobilized at different positions on the same surface on the same substrate and this is made to be a test area. Next, a test sample (or an extract thereof) which may contain the test substance, and the binding substance-labeling fluorescent particles in which the first binding substance and the third binding substance are bound are brought into contact with the substrate. If the test substance is not present in the test sample, reaction occurs on the substrate between the first binding substance which binds to the binding substance-labeling fluorescent particles, and the test substance itself which is capable of binding to the first binding substance immobilized on the test area or an analogous compound having the same epitope for the antibody of the first binding substance as the test substance. On the other hand, if the test substance is present, the reaction with the test substance itself which is capable of binding to the first binding substance or a compound having a similar site to the test substance and having the same epitope for the antibody of the first binding substance as the test substance is inhibited since the test substance binds to the first binding substance, the binding substance-labeling fluorescent particles are not immobilized on the test area. Meanwhile, on the control area, reaction occurs between the third binding substance on the binding substance-labeling fluorescent particles and the second binding substance immobilized on the control area, and the binding substance-labeling fluorescent particles are immobilized on the control area.

In the competitive method, a plurality of samples of the test substance of known amount with different test substance concentrations are prepared in advance, and the fluorescence signal of the control area and the test area is measured at several different times while this sample and the binding substance-labeling fluorescent particles come into contact on the control area and on the test area. From the results of these multiple measurements, the amount of fluorescence change over time (slope) is determined in each test substance concentration. The results are plotted with this change over time being made to be an Y axis and the concentration of the test substance to be an X axis, and a calibration curve of the concentration of the test substance for the amount of fluorescence change over time is obtained using a fitting method suitably matched such as a least squares method. The amount of the test substance included in the test sample may be quantified from the results of the amount of fluorescence change over time using a target test sample, based on the calibration curve obtained described above.

(Analogous Compound of Test Substance Having Epitope)

In the present invention, as the analogous compound of the test substance having an epitope which can be used in the competition method, an analogous compound of the test substance having an epitope for the first binding substance of the present invention, and for example, a hapten of the test substance (cortisol, or the like) may be preferably used.

(Substrate)

As the substrate when using an SPF method described later, using a substrate having a metal film on the surface is preferable. As the metal constituting the metal film, substances which can produce surface plasmon resonance may be used. Free-electron metals such as gold, silver, copper, aluminum or platinum may be preferably included, and gold is particularly preferable. These metals may be used either alone or as a combination. In addition, an intermediate layer made of chromium or the like between the layers made of a substrate and metal may also be provided considering the adhesion to the substrate. The thickness of the metal film is arbitrary, however, for example, greater than or equal to 0.1 nm and less than or equal to 500 nm is preferable, and greater than or equal to 1 nm and less than or equal to 200 nm is particularly preferable. If the thickness is above 500 nm, a surface plasmon phenomenon of a medium may not be sufficiently detected. Furthermore, when the intermediate layer made of chromium or the like is provided, the thickness of the intermediate layers is preferably greater than or equal to 0.1 nm and less than or equal to 10 nm.

Formation of the metal film may be carried out using conventional methods, and for example, a sputtering method, a vapor deposition method, an ion plating method, an electroplating method, an electroless plating method, or the like, may be used.

The metal film is preferably disposed on a base substrate. Here, "disposed on a base substrate" includes a case in which the metal film is disposed through other layers without being in direct contact with the base, in addition to a case in which the metal film is disposed to be in direct contact on the base. The base which may be used in the present invention may include, when considering the use for surface plasmon resonance biosensors, optical glass such as BK7 (borosilicate glass) which is one type of general optical glass, or a synthetic resin, specifically, those made of materials transparent to laser light such as polymethylmethacrylate, polyethyleneterephthalate, polycarbonate or cycloolefin polymer. The substrate such as this is preferably a material which does not show anisotropy for polarized light and has an excellent workability.

One example of the substrate for fluorescence detection using the SPF method may include a substrate in which polymethylmethacrylate (PMMA) is made to be a base and a gold film is vapor deposited.

(Detection Method of Fluorescence)

In the detection method of fluorescence in the present invention, detecting the fluorescence intensity using, for example, equipment capable of detecting the fluorescence intensity such as a microplate reader, a biosensor for fluorescence detection method using surface plasmon excitation (an SPF method), or the like, is preferable. The detection of the fluorescence intensity is usually completed after a certain period of time, for example, a few minutes to several hours, after the antigen-antibody reaction. The concentration of the test substance may be quantified from the relationship between the fluorescence intensity and the concentration of the test substance by detecting the degree of immune complex formation described above as the fluorescence intensity. In addition, the form of the measurement of fluorescence may be a plate reader measurement or a flow measurement. Furthermore, by using the SPF method, measurements with higher sensitivity than in using the fluorescence detection method by epi excitation (hereinafter, referred to as an "epi-fluorescence method") are possible.

As the surface plasmon fluorescent (SPF) biosensor described above, for example, a sensor disclosed in JP-2008-249361A, which includes an optical waveguide formed from materials which transmit excitation light of a predetermined wavelength, a metal film formed on one surface of this optical waveguide, a light source which generates a light beam, an optical system which generates a surface plasmon on the interface between the optical waveguide and the metal film by allowing the above light beam to pass through the optical waveguide and inputs the surface plasmon at an incident angle, and fluorescence detection means detecting the fluorescence generated from excitation by an evanescent wave enhanced by the surface plasmon, may be used.

(Method of detecting Test Substance Amount)

As one example of the quantifying methods of the test substance in the SPF method of the present invention, the test substance may be quantified using the following method. Specifically, samples containing each test substance of known concentration are prepared, and the fluorescence signal from the fluorescence detection site is measured at several different times while flowing down the fluorescence detection site. From the results of this multiple measurements, the amount of the fluorescence change over time (slope) is determined in each test substance concentration. The results are plotted with this change over time being made to be an Y axis and the concentration of the test substance to be an X axis, and a calibration curve of the concentration of the test substance for the amount of the fluorescence change over time is obtained using a fitting method suitably matched such as a least squares method. In an optical signal system, the amount of the test substance included in the target test sample may be specified based on the calibration curve corresponding to each test substance.

The fluorescence detection system by surface plasmon excitation (SPF) using the fluorescent particles of the present invention is an assay method detecting the fluorescence from the fluorescent substance depending on the amount of the test substance immobilized on the thin metal film on the substrate, and is different from a method in which changes in optical transparency are detected, for example, as turbidity, by the progress of a reaction in solution, which is so-called a latex agglutination method. In the latex agglutination method, an antibody-sensitized latex in a latex reagent and an antigen in an analyte are bound by an antibody reaction, and aggregated. This aggregates increases with time, and a method quantifying the antigen concentration from the absorbance change per unit time obtained from the irradiation of near infrared light to these aggregates is the latex agglutination method. In the present invention, when compared to the latex agglutination method, a very simple detection method of the test substance may be provided.

The method of detecting the substance to be detected using the fluorescent particles of the present invention may be performed using a kit or a chip having two measurement areas of a test area and a control area on a substrate, and mounting the substrate in which the second binding substance capable of binding to the first binding substance which binds to the fluorescent particles is immobilized on the control area. The test area and the control area on the substrate are preferably composed of materials which generate surface plasmon resonance. In the kit or the chip for detecting the test substance, by contacting these areas with the test sample containing the test substance and the fluorescent particles, and by reacting the test substance and the fluorescent particles, or the test area and the fluorescent particles, and the control area and the fluorescent particles, the fluorescence intensity of the test area and the control area are measured, and the fluorescence signal value of the test area is corrected using the value of the fluorescence signal in the control area. In the kit or the chip for detecting the test substance, the fluorescent particles may be binding substance-labeling fluorescent particles in which (i) one or more of a first binding substance which can bind to the test substance, and (ii) a third binding substance which can bind to a second binding substance immobilized on the control area and which does not bind to the test substance are bound, and the second binding substance is immobilized on the control area. As the examples of each material and the preferable ranges that constitute the chip or the kit for detection of the test substance using the fluorescent particles may use the examples and the ranges described in the detection method of the test substance using the fluorescent particles.

The present invention will be described more specifically using the following examples, however, the present invention is not limited by the examples.

### Examples

<Comparative Example 1>

(Preparation of Carbodiimide Treated Fluorescent Latex Particles)

250 µL of 50 mM MES buffer solution (pH 6.0) was added to 250 µL of an aqueous solution of fluorescent latex particles with a solids concentration of 2% by mass (a product by Life Technologies Corporation (formerly Invitrogen Corporation), an average particle diameter of 200 nm), and after the solids concentration was made to be 1% by mass, 8 µL of 10 mg/mL aqueous solution of EDC (1-ethyl-3-(3-dimethylaminopropyl)carbodiimide hydrochloride, product number 01-62-0011, Wako Pure Chemical Industries, Ltd.) was added and stirred at room temperature (1,200 rpm, 25°C 15 minutes), and carbodiimide treated fluorescent latex particles were obtained.

(Preparation of Anti-cortisol Antibody Binding Fluorescent Latex Particles)

9.5 µL of an anti-cortisol antibody (hereinafter referred to as an anti-COR antibody, manufactured by Hytest Ltd., product name: Anti-Cortisol MAb XM210, 6.3 mg/mL) was added as a first binding substance to 500 µL of the carbodiimide treated fluorescent latex particles, and the mixture was stirred at room temperature (1,200 rpm, 25°C 2 hours). After 25 µL of 2 mol/L aqueous solution of glycine (Wako Pure Chemical Industries, Ltd.) was added and stirred for 30 minutes, fluorescent latex particles were precipitated using a centrifuge (15,000 rpm, 4°C, 15 minutes). The supernatant solution was removed, 500 µL of PBS solution (phosphate buffer solution, Wako Pure Chemical Industries, Ltd., pH 7.4) was added, and the fluorescent latex particles were re-dispersed using an ultrasonic cleaning machine. Further centrifugation (15,000 rpm, 4°C, 15 minutes) was carried out, the supernatant was removed, and then 500 µL of PBS solution (pH 7.4) including 1% by mass of BSA (bovine serum albumin, Wako Pure Chemical Industries, Ltd.) was added, and after the fluorescent latex particles were re-dispersed, a solution of 1% by mass anti-COR antibody binding fluorescent latex particles (reaction reagent) was obtained.

(Preparation of Substrate)

On one side of a substrate with polymethylmethacrylate (PMMA, manufactured by Mitsubishi Rayon Co., Ltd., Acrypet VH-001) as a base, gold used on a test area and an adjacent control area was deposited so that both the test area and the control area had a width of 4 mm and a thickness of 70 nm. This substrate was cut to have a width of 5 mm and the substrate was prepared. On the gold deposited film of the test area of this substrate, a solution (concentration: 50 µL/mL in 50 mM MES buffer solution pH 6, 150 mM NaCl) including cortisol hapten (manufactured by Fitzgerald Industries, 80-IC10) was spotted, incubated for 1 hour at 25°C, and was immobilized by physical adsorption. On the other hand, on the gold deposited film of the control area, a solution (concentration: 0.19, 0.75 and 6.0 µg/mL in PBS solution) including an anti-mouse antibody (Anti-mouse IgG F(ab')2, product name: AffiniPure F(ab')2 Fragment Rabbit Anti-mouse IgG (H + L) as the second binding substance, manufactured by Jackson Immuno Research Inc.) was spotted, incubated for 1 hour at 25°C, and was immobilized by physical adsorption.

(Cleaning of Substrate, Blocking)

Before mounting three types of substrates prepared as described above to the flow path of the sensor chip, the substrates were repeatedly cleaned 3 times using 300 µL of cleaning solution (PBS solution (pH 7.4) including 0.05% by mass of Tween 20 (polyoxyethylene (20) sorbitan monolaurate, Wako Pure Chemical Industries, Ltd.) prepared in advance. After the cleaning was completed, 300 µL of PBS solution (pH 7.4) including 1% by mass of casein (manufactured by Thermo Fisher Scientific Inc.) was added and was allowed to stand at room temperature for 1 hour to perform blocking for the anti-mouse antibody on the evaporated gold film and non-adsorbed part of the cortisol hapten. After cleaning with the above cleaning solution, 300 µL of an Immunoassay Stabilizer (manufactured by Life Technologies Corporation (formerly ABI)) was added as a stabilizing agent, was allowed to stand at room temperature for 30 minutes, and solution was removed and water was completely removed using a dryer.

(Preparation of Sensor Chip)

A flow path-type sensor chip was prepared by sealing the three types of substrates prepared on the flow path as the configuration of a second embodiment of JP2010-190880A.

(Measurement)

The serum of 3 µg/dL cortisol concentration of the test substance and the anti-COR antibody-labeling fluorescent latex particles were mixed in advance. Spotting was performed on the flow path-type sensor chip prepared under temperature conditions of 26°C, 30°C, and 34°C, respectively, and was allowed to flow at a rate of 10 µL/min while performing a pump suction. The fluorescence intensity of the test area and the control area at the time of flowing down was measured, and the coefficient of the intensity change rate of the fluorescence intensity signal per unit time at each temperature condition was determined. The rate of this coefficient being caused to fluctuate due to temperature changes was determined, and in the test area, the rate of the coefficient change rate due to temperature was confirmed to be 7%/1°C. On the other hand, for the control area, the rate of coefficient change due to temperature of the substrate prepared by spotting a solution with each anti-mouse antibody concentration of 0.19, 0.75 and 6.0 µg/mL was determined. The results are shown in Fig. 1. Even at any anti-mouse antibody concentration of the control area, the change rates of the fluorescence amount of signal intensities were all less than 4% per 1°C, therefore, a gap of the temperature characteristics with the test area was large.

<Example 1>

(Review of Temperature Characteristics Using Fluorescence Latex Particles Which Label Two Types of Antibodies)

9.5 µL of an anti-COR antibody (6.3 mg/mL) as a first binding substance and 54.7 µL of an anti-TSH antibody (5.48 mg/mL, product name: Mab to TSH, manufacturer: OEM CONCEPTS) as a third binding substance were added to 500 µL of the carbodiimide treated fluorescent latex particles prepared in Comparative Example, and the mixture was stirred (1,200 rpm) for 2 hours at 25°C. After 25 µL of 2 mol/L aqueous solution of glycine was added and stirred for 30 minutes, the mixture was centrifuged (15,000 rpm, 4°C, 15 minutes) and the fluorescent latex particles were precipitated. The supernatant was removed, 500 µL of PBS solution (pH 7.4) was added, and the fluorescent latex particles were re-dispersed using an ultrasonic cleaning machine. Further centrifugation (15,000 rpm, 4°C, 15 minutes) was carried out, the supernatant was removed, and then 500 µL of PBS solution (pH 7.4) including 1% by mass of BSA was added, and after the fluorescent latex particles were re-dispersed, a solution of 1% by mass anti-COR antibody binding fluorescent latex particles was obtained.

The preparation of the substrate, the blocking of the substrate, the preparation of the sensor chip, and the measurements were performed in the same manner as those in Comparative Example 1, and the same substrate was used for the detection of cortisol as the Comparative Example.

The measurement was performed in the same manner as that in Comparative Example. The rate of change of the coefficient of the fluorescence signal intensity due to temperature of the test area was checked, and it was confirmed that the concentration of the analyte changed by 7% for a temperature change of 1°C. In addition, the rate of change of the coefficient of the fluorescence signal intensity due to temperature using the substrate in which the amount of the anti-mouse antibody of the control area is changed to be the same as that in Comparative Example as shown in Table 2 was checked, and it was confirmed that the rate of signal change dependent of the analyte concentration was 7.5% per 1°C at the substrate antibody concentration of 0.19 µg/mL. The rates of change at other concentrations were 4% or less. From these measurement results, it was confirmed that providing an assay method is possible in which the signal information of the test area is corrected by the signal value in the control area and the temperature dependence is very small by setting the amount of the anti-mouse antibody to be optimal.

## Claims

1. A method of detecting a test substance, comprising:
(1) reacting fluorescent particles and the test substance by bringing the test substance and the fluorescent particles into contact with a test area and a control area on a substrate,
(2) measuring fluorescence of the fluorescent particles of the test area and the control area, and
(3) correcting a fluorescence signal value in the test area using a fluorescence signal value in the control area,
wherein the fluorescent particles are binding substance-labeling fluorescent particles in which (i) one or more of a first binding substance which can bind to the test substance, and (ii) a third binding substance which can bind to a second binding substance and does not bind to the test substance are bound, and
wherein the second binding substance which can bind to the first binding substance is immobilized on the control area.

2. The method for measuring a test substance according to Claim 1,
wherein the test substance or an analogous compound of the test substance having an epitope for the first binding substance is immobilized on the test area.

3. The method for measuring a test substance according to Claim 2,
wherein, in step (1), the test substance brought into contact with the substrate and the test substance or the analogous compound thereof immobilized on the test area of the substrate are made to compete for the binding substance-labeling fluorescent particles.

4. The method for measuring a test substance according to any one of Claim 1 to 3,
wherein the first binding substance is an antibody which can bind to the test substance, and the second binding substance immobilized on the control area is an antibody which can bind to the antibody which is the first binding substance and to the third binding substance which does not bind to the test substance, respectively.

5. The method for measuring a test substance according to any one of Claim 1 to 4,
wherein the third binding substance is an antibody which can bind to the second binding substance and does not bind to the test substance.

6. The method for measuring a test substance according to any one of Claim 1 to 5,
wherein the fluorescent particles are fluorescent latex particles.

7. The method for measuring a test substance according to any one of Claim 1 to 6,
wherein, in step (2), the fluorescence is measured using a fluorescence detection method by surface plasmon excitation or a fluorescence detection method by epi excitation.

8. A test substance measurement chip used in measuring a test substance, comprising:
a test area and a control area on a substrate,
wherein fluorescent particles and a test substance are reacted by bringing each area into contact with the test substance and a test sample containing the fluorescent particles, the fluorescence of the fluorescent particles of each area is measured, and a fluorescence signal value in the test area is corrected using a fluorescence signal value in the control area,
wherein the fluorescent particles are binding substance-labeling fluorescent particles in which (i) one or more of a first binding substance which can bind to the test substance, and (ii) a third binding substance which can bind to a second binding substance and which does not bind to the test substance are bound, and
wherein the second binding substance which can bind to the first binding substance is immobilized on the control area.

9. A test substance measurement kit, comprising:
a test substance measurement chip including a test area and a control area on a substrate and used in measuring a test substance by reacting fluorescent particles and the test substance by bringing each area into contact with the test substance and a test sample containing the fluorescent particles, measuring the fluorescence of the fluorescent particles of each area, and correcting a fluorescence signal value in the test area using a fluorescence signal value in the control area; and
fluorescent particles used for the test sample, and
wherein the fluorescent particles are binding substance-labeling fluorescent particles in which (i) one or more of a first binding substance which can bind to the test substance, and (ii) a third binding substance which can bind to a second binding substance and which does not bind to the test substance are bound, and
wherein the second binding substance which can bind to the first binding substance is immobilized on the control area.
